# EUROPEAN PATENT APPLICATION

(11) **EP 4 667 999 A1**
(43) Date of publication of application: **24.12.2025**
(21) Application number: 24183424.1
(22) Date of filing: 20.06.2024
(51) Int. Cl.: G02B 23/24, G02B 26/08, A61B 1/00, G02B 7/24

(54) **OPTICAL SYSTEM**

(71) Applicant: Fisba AG, 9016 St. Gallen (CH)
(72) Inventor: Rehn, Henning, 9016 St. Gallen (CH)
(74) Representative: Hepp Wenger Ryffel AG

(57) **Abstract**

The invention relates to an optical system (10), in particular for a long and slim optical image transmission system, for example an endoscope, and an optical image transmission system.

The optical system (10) comprises an entrance lens unit (20), with at least one entrance lens, and an objective lens system (30), comprising at least one imaging lens. The optical system (10) further comprises a, in particular exactly one, deflection element (2) having a, in particular exactly one, reflecting plane (3). The deflection element (2) comprises an imaging body (4) and the reflecting plane (3) is part of surface of the imaging body (4), wherein the imaging body (4) is arranged in the optical path (P) between the entrance lens unit (20) and the objective lens system (30).

## Description

The invention relates to an optical system, in particular for a long and slim optical image transmission system, for example an endoscope, and an optical image transmission system.

Long and slender optical image transmission systems, for example endoscopes, are widely used to examine and diagnose intracavi-tary organs, intra-organic wall surfaces or other targets by inserting a tubular insert into a body cavity. Endoscopes are also used in the industrial sector to inspect the inside of pipes, boilers, machines, chemical plants, etc.

Such image transmission systems are usually accompanied by an optical illumination system for emitting illumination light on the distal side of the image transmission system.

Such an image transmission system can have a flexible or a rigid tubular housing.

Simple image transmission systems are realized with a field of view centred around the system axis (the mechanical axis of the tubular housing). However, many applications require an oblique direction of view, for example for enlarging the viewing angle. In this case, the image transmission system preferably has a single or compound prism (such as described in US4138192A) near the distal end so that the optical axis of the distal opening forms an angle with the system axis.

For an image transmission system inside a flexible, tubular housing, the system axis is understood to be the direction of the distal end of the bent housing.

The image transmission system of a rigid endoscope usually comprises a, preferably concave, entrance lens, a deflection element, an objective lens system, a relay system consisting of a plurality of rod lenses, and an eyepiece on the proximal side of the image transmission system, i.e. on the observer's side.

Another type is a semi-flexible endoscope with a chip-on-tip optics that features a concave entrance lens, a deflection element, an objective lens system and an image sensor within the flexible tip of an endoscope. Such a system does not have any eyepiece and no way for direct human observation, any image is recorded within the tip and transmitted electronically.

A prism is usually used as the deflection element to provide a viewing direction different from the system axis.

Typically, such prisms feature two reflections, to keep the system compact and to produce an upright image when looking into the eyepiece. The prism can be formed by a single element or by an assembly of several prisms. The latter form is preferred for mechanical stability, easy handling, and the option to attach the concave entrance lens, but related to high effort and cost in manufacturing.

WO 2021/032340 A1 discloses a long and slim optical image transmission system with an optical system comprising a prism having two reflecting planes.

The optical design of the image transmission system of a rigid or semi-flexible endoscope would benefit from a system aperture position within the prism which is hard or impossible to realize. This design advantage would be related to less aberrations, in particular distortion, and would allow for the use of fewer lens elements. Instead, and as a compromise, the system aperture is often placed after the prism body with respect to the direction of incoming light.

It is the task of the invention to present an optical system and an optical image transmission system comprising a novel deflection element which avoid some disadvantages of the known elements and, in particular, permit easy assembly and good manageability with precise imaging properties.

The problem is solved by the features of the independent claims.

An optical system, in particular for a long and slim optical image transmission system, for example an endoscope, comprises an entrance lens unit, an objective lens system and further a, in particular exactly one, deflection element.

The entrance lens unit comprises at least one entrance lens, preferably with a negative focal length, and the objective lens system comprises at least one imaging lens. The entrance lens may have a plano-concave, a concave-concave or a concave-convex surface.

The deflection element has a, in particular exactly one, reflecting plane.

The deflection element comprises an imaging body and the reflecting plane is part of surface of the imaging body. The imaging body is arranged in the optical path between the entrance lens unit and the objective lens system.

To reach the reflecting plane, light has to cross the imaging body.

Within this context an imaging body comprises a transparent, but dielectric volume with an entrance surface and an exit surface. At least one of the surfaces is curved, preferably both.

Thus, the deflection element comprises a transparent, but dielectric, volume with an entrance surface, an exit surface, and a reflecting surface, the reflecting surface preferably flat.

Along the optical path from the entrance lens unit to the objective lens system the suggested deflection element has an entrance surface, an exit surface and a reflecting surface in between.

Preferably, the deflection element is a single body of a dielectric such as an optical glass, that comprises a, preferably flat, reflection surface. The deflection element may be used in a similar way as a side view prism.

The deflection element preferably is arranged such that the normal direction of the flat reflecting surface is the bisector of the system axis and the optical axis of the entrance lens.

The entrance surface and the exit surface may be formed by surface portions of a circular cylinder. The imaging body may be realized by a half cylinder lens, having a cylindric body with a semi-spherical base area and a rectangular reflecting plane. The deflection element in this case may be combined with at least one or more anamorphic lens in the objective lens system, to enable a specific (desired) aspect ratio of the image different of the object.

The imaging body comprises an entrance surface and an exit surface which each may be formed by a surface portion of a sphere.

Preferably, the imaging body is a hemisphere, also called a half ball lens.

The hemisphere or half ball lens comprises a circular reflecting plane.

The refractive power introduced this way improves the system performance and may even allow for less lens elements in the objective lens system, compared to other deflection elements.

The hemisphere may have a radius from 0.5mm to 5mm, such that it can be used in slim optical image transmission systems, such as endoscopes for medical or technical use.

Preferably, the spherical centre of the hemisphere is crossed by both the optical axis of the entrance lens and the system axis.

The imaging body may be formed by a part of a hemisphere, wherein parts not contributing to light transmission, especially for specific viewing angles, are cut out. For example, a cylindrical cut of a hemisphere may be used.

This may facilitate mounting and may save space and weight.

The non-hemispheric part of the imaging body may be used for fixing or framing the imaging body, for example in a frame or rim.

The imaging body preferably is made of optical glass, for example N-BK7 or N-BAK4. The optical glass may have a refractive index from 1.42 to 1.72.

It turned out that a refractive index of 1.45 to 1.72 is preferred, in contrast to a refractive index larger than 1.9 often used for prisms. This enables easier and more efficient coatings such as for the reduction of unwanted reflections.

The reflecting surface may work by total internal reflection or the deflection element may comprise a reflective coating on the flat surface of the imaging body.

As a result of the hemispheric or nearly hemispheric shape of the imaging body, the system aperture can be arranged between its entrance and exit surfaces of the imaging body.

An aperture may for example be applied on the flat surface of the imaging body. As compared to systems with side view prisms, this arrangement of the aperture means an arrangement more upstream with respect to the incoming light. An aperture arranged more upstream in the light path allows for a better imaging quality with a given number of lenses. The distortion usually is dependent on the position of aperture in the light path, wherein a position more upstream results in a lower distortion.

If the hemispheric lens works by total internal reflection (TIR) due to a high refractive index, the system aperture can be realized by coating the outer regions of the reflecting surface with a black paint leaving the inner part for TIR. Alternatively, the outer regions may be roughened to avoid specular reflection.

Half spheres without TIR can carry a reflective coating (metallic or dielectric) at least in the centre to realize the system aperture.

The shape of this region can be preferably circular, elliptic or oval.

Alternatively, the system aperture can be realized by an aperture element that would carry the region with the reflective coating and would be cemented to the flat surface of the imaging body. Such a mechanical body with the aperture could also be an aid in mounting the half sphere lens.

In contrast to a prism that can be possibly glued to a first lens element, the optical system may comprise a mechanical lens mount to hold the deflection element and preferably the entrance lens unit. The lens mount may fix the deflection element and preferably the entrance lens unit to the housing of the optical system and/or the objective lens system and may provide for a specific viewing angle.

Different lens mounts may be used for different viewing directions, realized with one and the same set of optical components. The user may choose between the mechanical systems depending on the desired viewing angle.

The lens mount may be adjustable to compensate for aberrations elsewhere in the system.

An arrangement that allows for the change of the viewing direction during operation can be conceived.

The optical system may comprise a rotating mechanism for rotating the deflection element around an axis within the reflecting plane and perpendicular to the system axis and the viewing direction.

The optical system may comprise a rotating mechanism for rotating the entrance lens, wherein the rotating mechanism for the deflection element and the rotating mechanism for the entrance lens unit are coupled, in order to redirect the optical axis of the entrance lens unit by an angle twice as large as that of the deflection element.

In particular, the half ball lens may rotate with half the angular velocity of the front lens axis, enabled by a suitable mechanical setup. This can be realized by the optical system comprising respective electronic and/or mechanical means.

The optical system may comprise a driving element, for example a motor, for moving the entrance lens unit and/or the deflection element.

According to a further aspect an image transmission system, in particular a long and slim optical image transmission system, for example an endoscope, having a system axis, comprises a tubular housing and comprises at least one optical system as described above mountable or mounted to the tubular housing.

The optical system may be firmly attached to the tubular housing, or at least a part of the optical system may be removably attached to the housing. Before use, the optical system or the part of the optical system may be mounted to the tubular housing by a user or by a provider.

A sub-system may at least comprise the entrance lens unit and the imaging body, mounted to a sub-system housing, for example having also a tubular form, for a specific viewing angle.

The sub-system may also comprise an aperture, the objective lens system and/or an image sensor.

The sub-system may be realized as a drop-in assembly and may be mountable to the tubular housing by a snap fit connection, by a screw connection, by clamp connection or by a bayonet fastener.

Alternatively, the image transmission system may comprise a mechanical and/or electronical control element for a rotation mechanism of the optical system to adjust the viewing angle for the same optical system.

An optical axis of the entrance lens unit, which defines the main viewing direction, may have a viewing angle with the system axis of 15° to 170°. Accordingly, a normal vector of the reflecting plane may include an angle between 82.5 to 5°, preferably of 75° to 60°, with the system axis of the long and slim optical image transmission system.

Thus, the optical system allows a wide viewing angle, including a backward view without moving the image transmission system.

For a chip-on-tip endoscope, the image transmission system may contain an image sensor, located downstream the entrance lens, the deflection element and the objective lens system with respect to the entering light.

The optical system preferably provides only one reflection. The image therefore is upside down. When received by an image sensor, this is irrelevant, because for displaying the image may electronically be converted, in particular digitally flipped and/or reverted.

A kit for an image transmission system as described above may comprise at least two sub-systems and at least one tubular housing, wherein each sub-system comprises at least an entrance lens unit and a deflection element.

The sub-system may comprise an optical system as described above.

Each sub-system may be mountable to the tubular housing, such that the tubular housing and the mounted sub-system comprise an optical system as described above.

The objective lens system and/or an image sensor may be mounted to the sub-system or to the tubular housing.

Each sub-system provides a specific different viewing angle when mounted to the tubular housing.

Thus, with the kit provides for an image transmission system with exchangeable sub-systems. A user can choose between sub-systems with different viewing angles.

Additionally, the kit may comprise a sub-system with a lens system providing a straight view.

For the realization of a straight view version with rather the same set of optical parts and a slightly different mechanical housing, the half sphere can be replaced by a full sphere of the same size. Alternatively, two half spheres can be glued together with the system aperture realized as an open region in the center of an otherwise blackened surface.

According to a further aspect, a deflection element is used as a light deflecting element in an optical system of an image transmission system. The deflection element comprises an imaging body and the surface of the imaging body has a, in particular exactly one, reflecting plane.

The image transmission system in particular is a long and slim optical image transmission system, for example an endoscope.

The deflection element is used, such that light entering an entrance lens unit enters a, preferably curved, entrance surface of the deflection element, is reflected at a, preferably flat, reflecting plane, crosses a, preferably curved, exit surface of the deflection element and enters an objective lens system of the optical system. Preferably, the used deflection element is a hemisphere or half-ball lens, as described above.

Preferred embodiments of the invention are explained in more detail in the following description with reference to the accompanying drawings. Corresponding elements are provided with matching reference signs.

It shows
- Figure 1: a schematical drawing of a first example of an op-tical system;
- Figure 2: a schematical drawing of a second example of an optical system with a first viewing angle;
- Figure 3: a schematical drawing of a third example of an op-tical system with a second viewing angle;
- Figure 4: a schematical drawing of a fourth example of an optical system with a third viewing angle;
- Figure 5a, 5b: a schematical drawing of a first example of an im-aging body in a sectional view and from below;
- Figure 6: a schematical drawing of a second example of an imaging body;
- Figure 7: a schematical drawing of a third example of an im-aging body;
- Figure 8: a schematical drawing of an example of an endo-scope;
- Figure 9: a schematical drawing of a further example of an optical system.

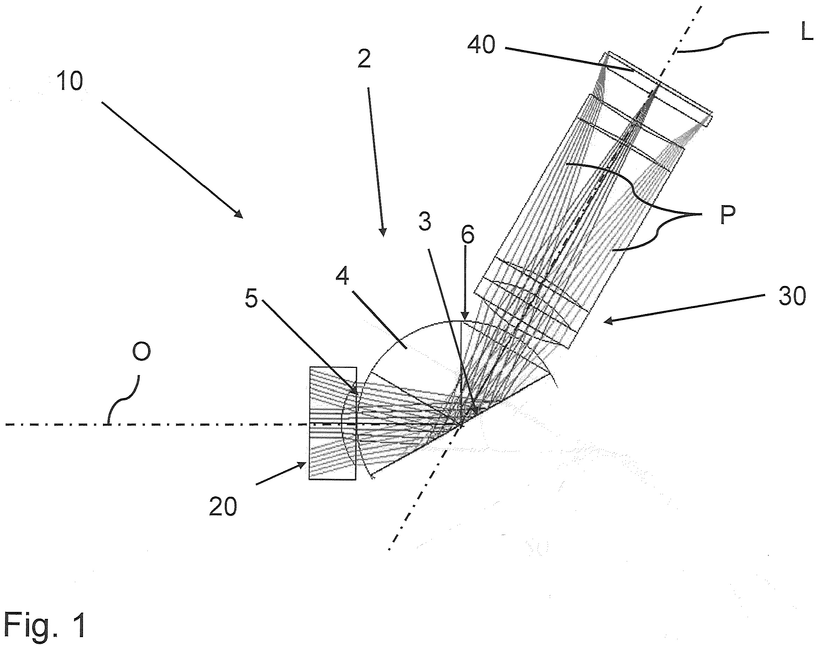

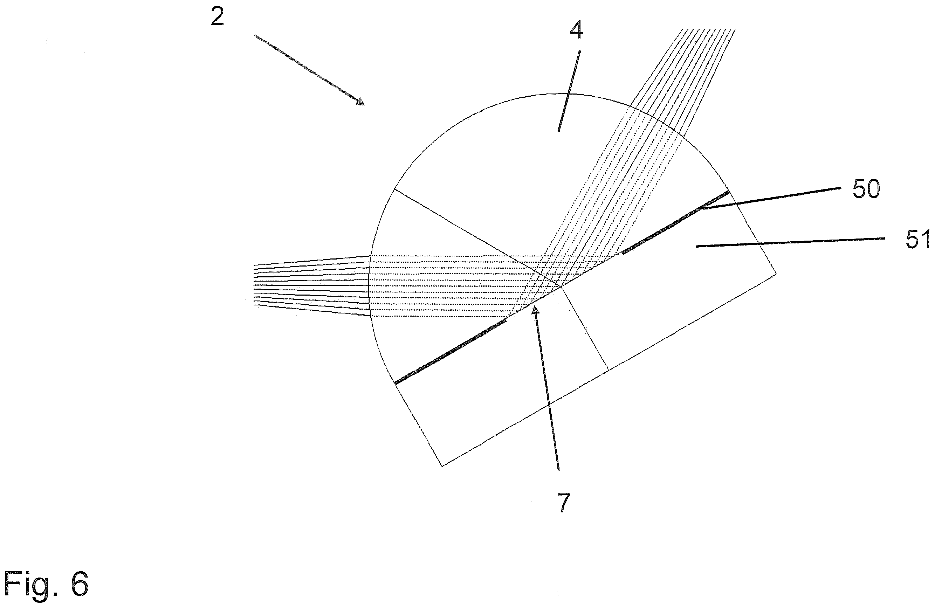

Figure 1 shows a schematical drawing of a first example of an optical system 10, in particular for a long and slim optical image transmission system 100 (see figure 6).

The optical system 10 comprises an entrance lens unit 20 and an objective lens system 30. A hemisphere is arranged as deflection element 2 in the optical path P between the entrance lens unit 20 and the objective lens system 30.

The deflection element 2 comprises a circular reflecting plane 3, which is part of surface of the hemispherical imaging body 4.

The optical axis O of the entrance lens unit 20 crosses the optical axis of the objective lens system 30, which usually coincide with the system axis L of the slim optical image transmission system 100 (see figure 6), in the reflecting plane 3.

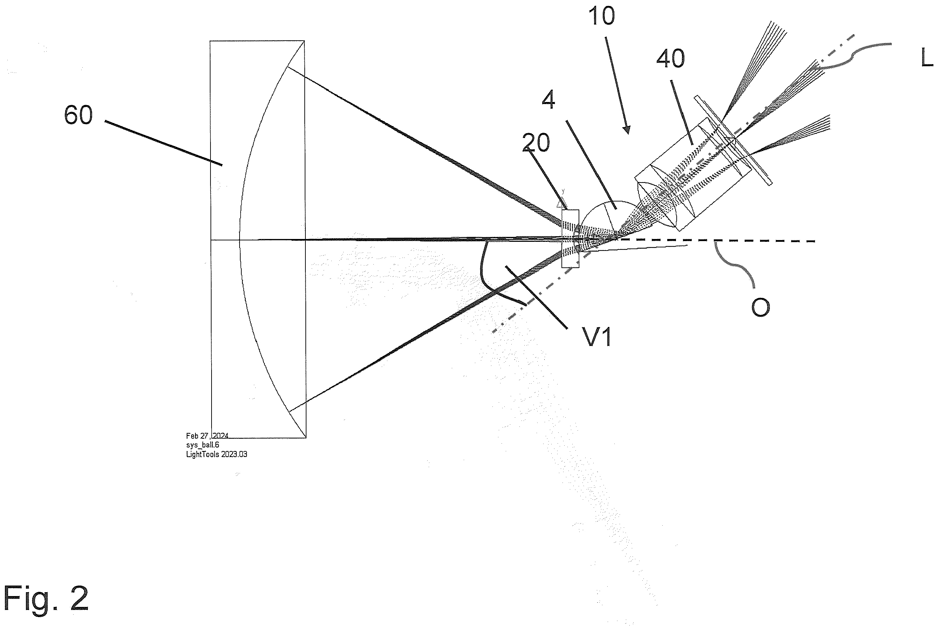

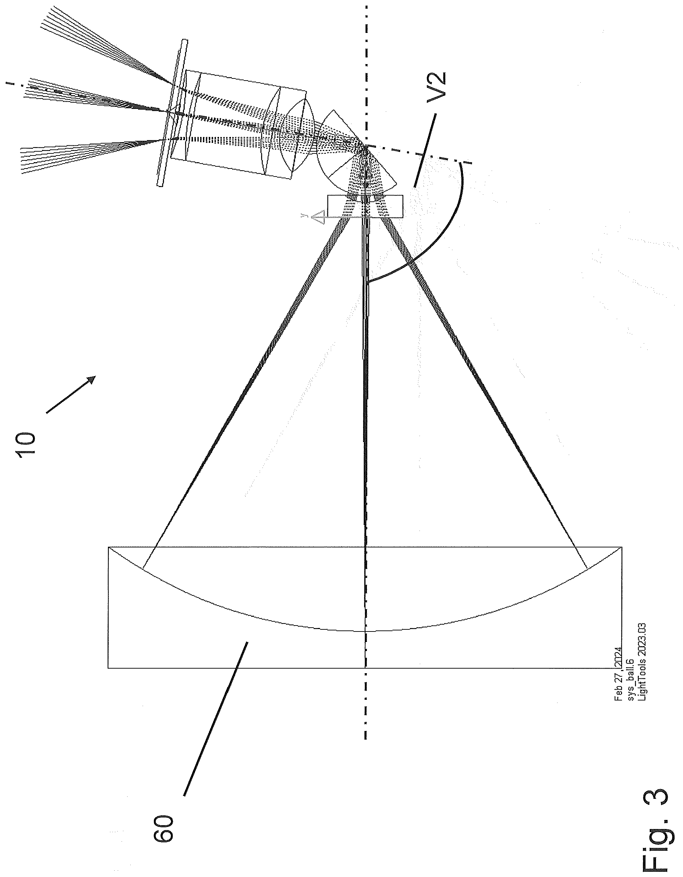

Figures 2-4 show schematical drawings of a second example of an optical system 10 with a first, second and third viewing angle V1; V2; V3.

The viewing angle V1; V2; V3 is the angle between the optical axis O of the entrance lens unit 20 and the system axis L. An object 60 to be imaged may be positioned in the optical axis O.

The viewing angle V1 is limited on the lower side by the possible field of view, which may be considered as the possible range of incoming light around viewing angle. As light has to be reflected at the deflection element 2, the field of view is given by the available reflection angles.

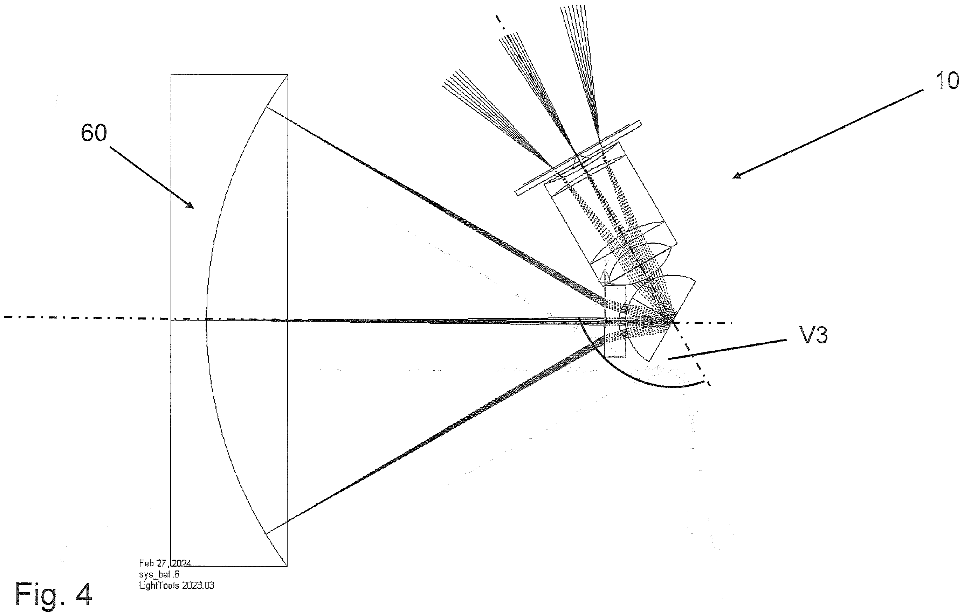

The viewing angle V3 is limited in the upper side by the spatial extension of the optical elements. Nevertheless, a backward view (V3 > 90°) is possible as can be seen in Figure 4 in contrast to prism arrangements.

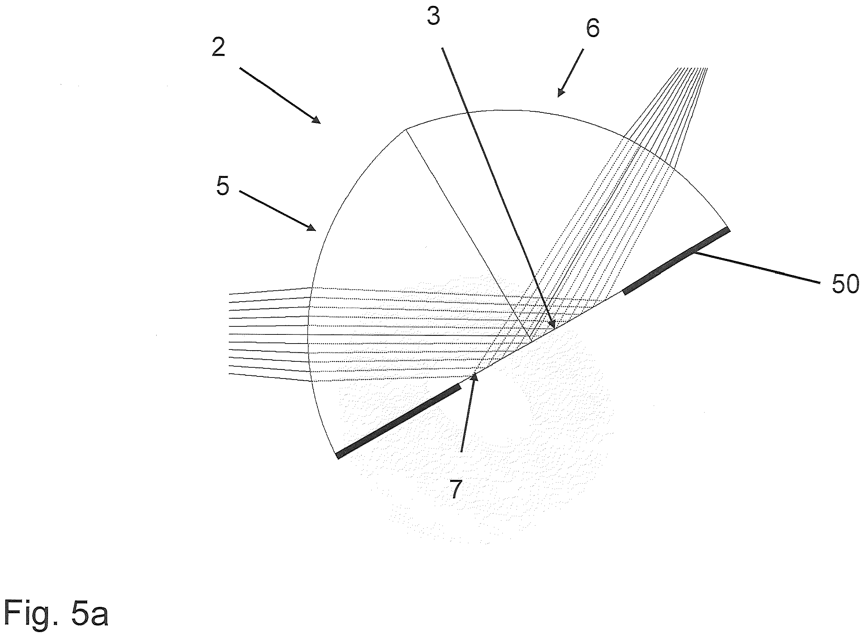

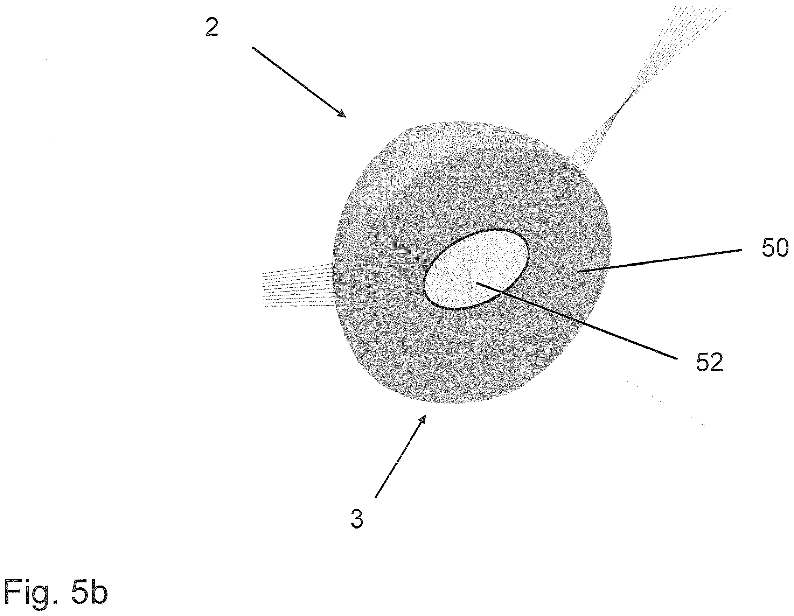

Figure 5a and 5b show a schematical drawing of a first example of a deflection element 2. The deflection element 2 comprises an imaging body 4 with an entrance surface 5 and an exit surface 6, which each are formed by a surface portion of a sphere. As the imaging body does nevertheless not have the form of a hemisphere, the shape of the reflecting plane 3 is not circular.

An aperture 50 is arranged on the outside of the reflecting plane 3. The aperture 50 may have been applied by coating or by printing on the flat surface 7 of the imaging body 4.

The aperture 50 has an aperture opening 52 with an oval shape. The aperture opening 52 may be filled with a reflective coating.

Figure 6 shows a schematical drawing of a second example of a deflection element 2. An aperture element 51 having an aperture 50 carries the deflection element 2. The aperture element 51 is cemented to the flat surface 7 of the imaging body 4.

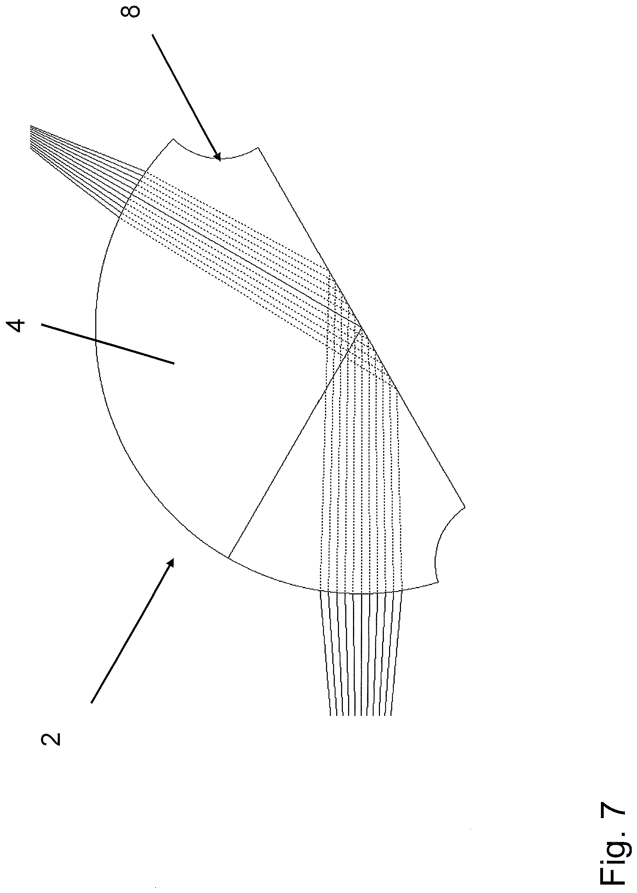

Figure 7 shows a schematical drawing of a third example of a deflection element 2. In this example, the imaging body 4 is formed by a part of a hemisphere, wherein parts not contributing to light transmission, are cut out.

This way, the deflection element 2 comprises a mounting surface 8, which may for example be annular. The mounting surface 8 may be mounted to a receiving surface of a mounting structure in a housing (not shown in the figure).

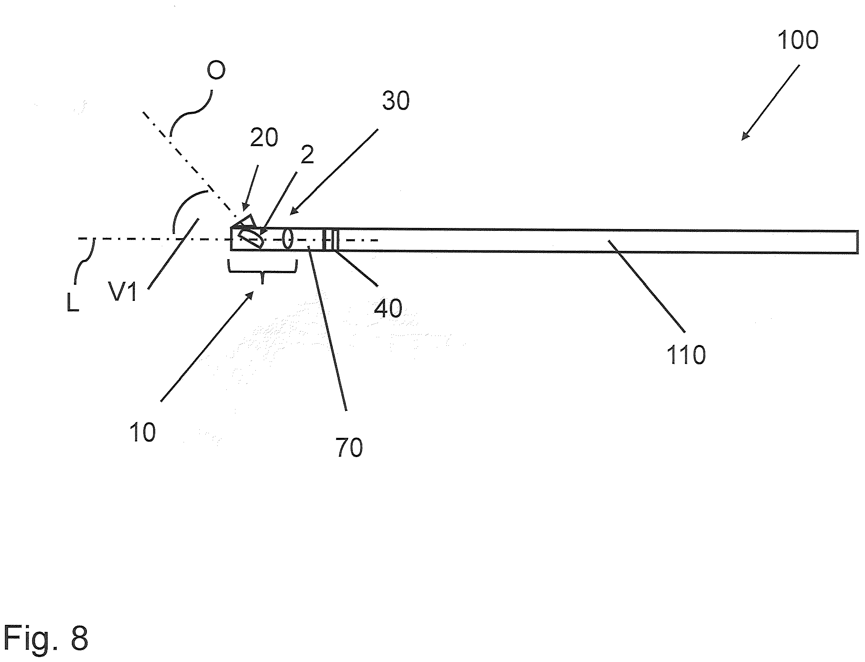

Figure 8 shows a schematical drawing of an example of an endoscope 100.

The endoscope 100 comprises an optical system 10 and a tubular housing 110 with a system axis L.

The optical system 10 comprises an entrance lens unit 20, an objective lens system 30 and deflection element 2.

The endoscope 100 comprises an image sensor 40.

The deflection element 2 is arranged to provide a viewing angle V1 between the optical axis L of the entrance lens unit 20 and the system axis L.

The optical system 10 is part of a sub-system 70 which is releasably fixed to the tubular housing 110.

Sub-systems 70 with different viewing optical systems 10 having different viewing angles may each be attached to the tubular housing 110.

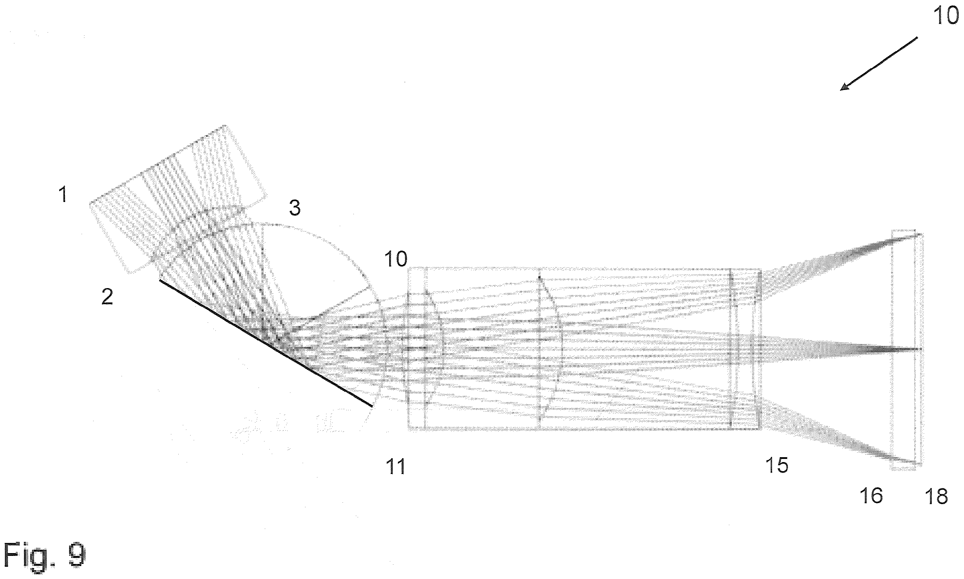

Figure 9 shows a schematical drawing of a further detailed example of an optical system 2.

The figure shows numbered surfaces of optical elements of the optical system 2, which correspond to the following table.

| # | Type | Radius | Thickness | Material | Semi-Diameter | Mech Semi-Dia | X tilt |
|---|---|---|---|---|---|---|---|
| 0 | STANDARD | 20.00 | 20.00 | | 10.28 | 10.28 | |
| 1 | STANDARD | Infinity | 0.40 | SAPPHIRE | 0.51 | 0.51 | |
| 2 | STANDARD | 0.56 | 0.16 | | 0.35 | 0.35 | |
| 3 | STANDARD | 0.80 | 0.80 | N-ZK7A | 0.80 | 0.80 | |
| 4 | COORDBRK | Infinity | 0.00 | | | | -30.00 |
| 5 | COORDBRK | Infinity | 0.00 | | | | 90.00 |
| 6 | STANDARD | Infinity | 0.00 | MIRROR | 0.63 | 0.63 | |
| 7 | COORDBRK | Infinity | 0.00 | | | | 90.00 |
| 8 | COORDBRK | Infinity | 0.00 | | | | -30.00 |
| 9 | STANDARD | Infinity | -0.80 | N-ZK7A | 0.25 | 0.80 | |
| 10 | STANDARD | 0.80 | -0.10 | | 0.80 | 0.80 | |
| 11 | STANDARD | -1.68 | -0.27 | N-SK14 | 0.37 | 0.52 | |
| 12 | STANDARD | 0.64 | -0.76 | N-LASF46B | 0.37 | 0.52 | |
| 13 | STANDARD | 0.79 | -1.13 | N-SSK2 | 0.46 | 0.52 | |
| 14 | STANDARD | 2.45 | -0.09 | N-SF11 | 0.51 | 0.52 | |
| 15 | STANDARD | -2.45 | -0.90 | | 0.52 | 0.52 | |
| 16 | STANDARD | Infinity | -0.15 | N-BK7 | 0.74 | 0.77 | |
| 17 | STANDARD | Infinity | -0.05 | | 0.77 | 0.77 | |
| 18 | STANDARD | Infinity | 0.00 | | 0.75 | 0.75 | |

Lengths are given in mm.

## Claims

1. Optical system (10), in particular for a long and slim optical image transmission system, for example an endoscope (100),
comprising an entrance lens unit (20), comprising at least one entrance lens, and an objective lens system (30), comprising at least one imaging lens,
further comprising a, in particular exactly one, deflection element (2) having a, in particular exactly one, reflecting plane (3),
**characterized in that** the deflection element (2) comprises an imaging body (4) and the reflecting plane (3) is part of surface of the imaging body (4), the imaging body (4) being arranged in the optical path (P) between the entrance lens unit (20) and the objective lens system (30).

2. Optical system (10) according to claim, wherein the imaging body (4) comprises an entrance surface (5) and an exit surface (6) which each are formed by a surface portion of a sphere.

3. Optical system (10) according to claim, wherein the imaging body (4) is a hemisphere.

4. Optical system (10) according to one of the preceding claims, wherein the imaging body (4) is made of optical glass, for example N-BK7 or N-BAK4, preferably with a refractive index n=1.42 to n=1.72.

5. Optical system (10) according to one of the preceding claims, wherein the optical system (10) comprises an aperture (50), preferably an aperture element (51), arranged at or adjacent to the reflecting plane (3), more preferably the aperture (50) having a reflective aperture opening (52) and/or and having an aperture opening (52) with an oval shape.

6. Optical system (10) according to one of the preceding claims, wherein the deflection element (2) comprises a reflective coating on the reflecting plane.

7. Optical system (10) according to one of the preceding claims, wherein the optical system (10) comprises a rotating mechanism for rotating the deflection element (2) around an axis within to reflecting plane (3) and perpendicular to the system axis and viewing direction.

8. Optical system (10) according to claim 7, wherein the optical system comprises a rotating mechanism for rotating the entrance lens (20), wherein the rotating mechanism for the deflection element (2) and the rotating mechanism for the entrance lens unit (20) are coupled, in order to rotate the entrance lens unit (20) by an angle twice as large as the deflection element (2).

9. Image transmission system (100), in particular a long and slim optical image transmission system, for example an endoscope, having a system axis (L), comprising a tubular housing (110) and comprising at least one optical system (10) according to any one of the preceding claims mountable or mounted to the tubular housing (110).

10. Image transmission system (100) according to claim 9,
wherein an optical axis (O) of the entrance lens unit (20) has a viewing angle (V, V1, V2, V3) with the system axis (L) of 15° to 170°.

11. Image transmission system (100) according to claim 9 or 10, wherein the image transmission system (100) comprises an image sensor (40).

12. Kit for an image transmission system (100) according to at least one of claims 9-11, comprising at least two sub-systems (70) and at least one tubular housing (110), each sub-system (70) comprising at least an entrance lens unit (20) and a deflection element (2), each sub-system (70) being mountable to the tubular housing (110), such that the tubular housing (110) and the mounted sub-system (70) together comprise an optical system (10) according to one of claims 1-8, wherein each sub-system (70) provides a different viewing angle (V, V1, V2, V3) when mounted to the tubular housing (110).

13. Use of a deflection element (2) comprising an imaging body (4), the surface of the imaging body having a, in particular exactly one, reflecting plane (3), as a light deflecting element in an optical system (10) of an image transmission system (100), in particular a long and slim optical image transmission system, for example an endoscope.
